Europäisches Patentamt

European Patent Office

Office européen des brevets

(19)

(11) EP 1 609 406 A1

(12) EUROPEAN PATENT APPLICATION

(43) Date of publication:
28.12.2005 Bulletin 2005/52

(51) Int Cl.⁷: **A61B 5/00**, A61B 10/00

(21) Application number: 05001807.6

(22) Date of filing: 28.01.2005

(84) Designated Contracting States:
AT BE BG CH CY CZ DE DK EE ES FI FR GB GR
HU IE IS IT LI LT LU MC NL PL PT RO SE SI SK TR
Designated Extension States:
AL BA HR LV MK YU

(30) Priority: 23.06.2004 JP 2004184407

(71) Applicant: Hitachi, Ltd.
Tokyo 100-8280 (JP)

(72) Inventor: Yamamoto, Tsuyoshi
Hitachi, Ltd. Int. Prop. Group
Chiyoda-ku Tokyo 100-8220 (JP)

(74) Representative: Strehl Schübel-Hopf & Partner
Maximilianstrasse 54
80538 München (DE)

(54) **Light measurement system for living body**

(57) Disclosed is a method for measuring light in a living body and capable of discriminating the size in activation brain areas. Two diamond-shaped probes alternately installed spatially for a light source or a light detector, and a second light source or light detector are installed between a first light source or light detector and an image is reconstructed utilizing all the sampling points, and whether or not the largest position on the reconstructed image is in proximity to the first or the second sampling point is determined, and an image is reconstructed from those results using only the position information from the first or the second sampling point. The difference in the sizes of the activation brain area can be displayed as an image, and an increased amount of information can be acquired from results measuring the metabolic substance concentration within a living body.

FIG.1

BLOOD VOLUME CHANGE AT EACH
SAMPLING POINTS (A-PLANE AND
B-PLANE) IS ESTIMATED.  1-1

BLOOD VOLUME AT EACH SAMPLING
POINTS ARE SPATIALLY INTERPOLATED.  1-2

LOCAL MAXIMUM PLACE OF THE BLOOD
VOLUME CHANGE DISTRIBUTION
IS DECIDED.  1-3

THE MAXIMAL
PLACE IS CLOSE
TO THE SAMPLING POINTS
OF THE A-PLANE OR
B-PLANE?  1-4

CLOSE TO THE
SAMPLING POINT
AT THE A-PLANE.

CLOSE TO THE
SAMPLING POINT
AT THE B-PLANE.

SAMPLING POINTS AT
A-PLANE ARE EMPLOYED
TO OBTAIN THE
TOPOGRAPHICAL IMAGE.  1-5

SAMPLING POINTS AT
B-PLANE ARE EMPLOYED
TO OBTAIN THE
TOPOGRAPHICAL IMAGE.  1-6

EP 1 609 406 A1

**Description**

CLAIM OF PRIORITY

The present application claims priority from Japanese application JP 2004-184407 filed on June 23, 2004, the content of which is hereby incorporated by reference into this application.

FIELD OF THE INVENTION

The present invention relates to a technology for measuring the concentration of metabolic substances within a living body by using light and showing the measurement results as images.

BACKGROUND OF THE INVENTION

Technology for measuring the metabolic substance concentration at multiple points within a living body by using light and then showing the measurement results as images are disclosed in the non-patent document 1 and elsewhere. The concept behind this technology is described as follows.

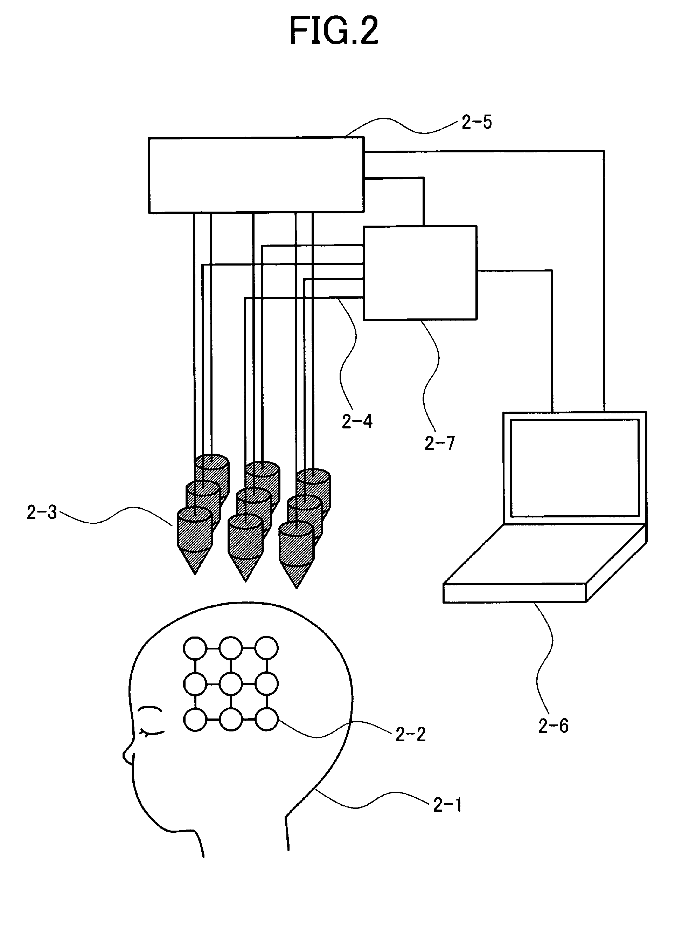

FIG. 2 is a concept diagram showing the structure of a device for implementing the biologic light measurement method. The measurement can be made in a state where a helmet 2-2 to which optical fibers for measurement are clamped as described later on, is placed on the scalp of the test subject 2-1. The optical fibers for measuring the light include optical fibers for light irradiation (2-3) for irradiating the light emitted from the light source onto the subject; and optical fibers for light detection (2-4) for collecting and transmitting to the light detector the light that permeated through the living body. The example in the present embodiment respectively uses 5 fibers (2-3) and 4 fibers (2-4). The tip of these light irradiation and light detection fibers contact (only) the scalp of the subject 2-1 and therefore the measurement can be made in a normal daily environment without harming the subject. These light irradiation fibers and light detection fibers are installed at alternate spatial intervals at intersections on a lattice. The usual gap between the nearest lattice intersections is preferably about 30 millimeters. The light irradiation fibers are connected to a light source array 2-5. Multiple light sources, typically semiconductor laser and light-emitting diodes comprise the light source array. The number of light sources is equivalent to the number of light irradiation fibers multiplied by the number of target substance types for measurement. A computer 2-6 controls the intensity of the light source. The light source arrays contain optical couplers and the light emitted from the multiple light sources of different wavelengths can irradiate into the respective light irradiation fibers. The light detection fibers for detecting light propagating through internal sections of the subject are connected to a light detector array 2-7 made up of light detectors typically comprised of photodiodes and photon multiplier tubes. The light sent to this light detector array is converted into electrical signals, and information on that light intensity is conveyed to the computer 2-6 in real-time. Besides controlling the intensity of the light source as described above, the computer also contains a function to calculate the fluctuation in metabolic substance concentrations within the living body based on fluctuations in light intensity, and a function to display an image and a time course of fluctuations in metabolic substance concentrations based on those calculated results.

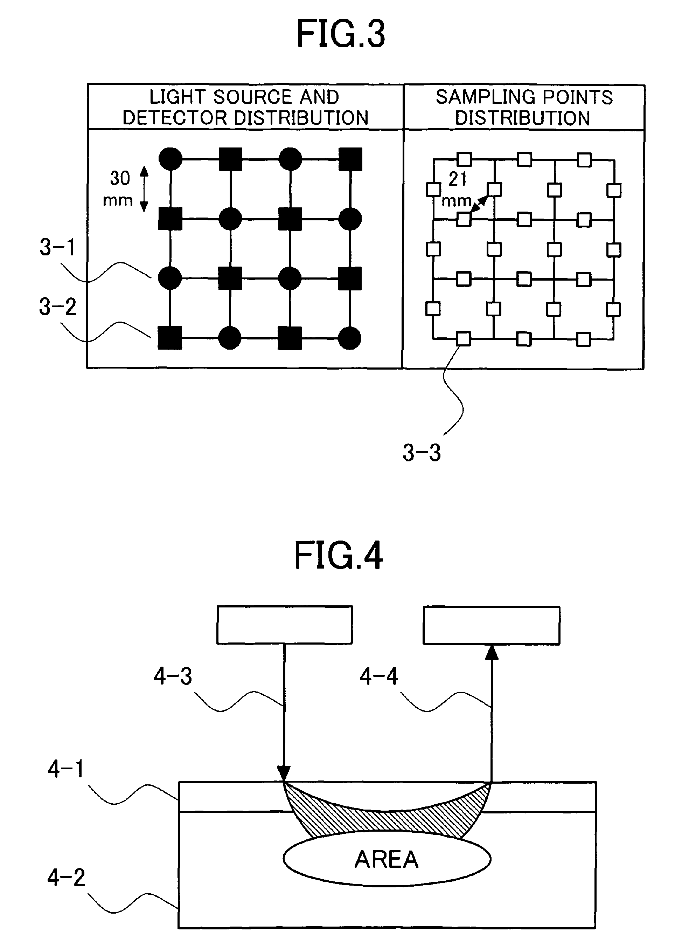

The clinical effectiveness of the technology shown in FIG. 2, as used for example by a neurosurgeon is disclosed in the non-patent document 2 and the non-patent document 3. This measurement technology differs greatly is some points from conventional technology for measuring brain functions such as fMRI (functional magnetic resonance imaging) and PET (positron emission tomography). In these devices such as fMRI and PET for measuring brain functions, the signals emitted from a living body can be measured without the measurement sensors making (actual) contact with the subject. Therefore many sampling points can be set at desired positions needed for imaging. The spatial resolution of the images is therefore high. However the light measurement system for a living body as shown in FIG. 2 utilizes optical fibers for measurement that make contact with the scalp of the patient. Though the optical fiber is well capable of following the movements of the subject, this method has the problem that spatially increasing the number of sampling points is difficult. The number of sampling points is essentially small compared to fMRI and PET so that the spatial resolution is low. The diagrams in FIG. 3 show the installation positions for the light irradiation fibers and light detection fibers in the light measurement system for a living body shown in FIG. 2, as well as the distribution of sampling points for supplying position information on fluctuations in the concentrations of metabolic substances within the body detected by the light irradiation fibers and light detection fibers. In this installation method, the light sources (sources 3-1 are shown as a dot in the figure) and the light detectors (detectors 3-2 are shown as a black square in the figure) are installed alternately at mutual spaced intervals at intersections within the lattice.

The installation positions in the figure are at fixed intervals of 30 millimeters. The sampling points 3-3 can consequently be distributed at equally spaced intervals of 21 millimeters. The reason is described while referring to FIG. 4. FIG. 4 shows the propagation path of the light emitted from a particular location. In this figure, 4-1 and 4-2 denote the typical skull and the cerebral cortex from the brain structure. The light emitted from the light irradiation fiber 4-3 is scattered due to the scattering (or diffusion) effect of body structures especially the skull. Installing a light detection fiber 4-4 at a location 30 millimeters away from the irradiation fiber 4-3 is known to propagate the light in a banana-shaped path shown in 4-5. When for example, measuring the changes in blood flow that accompany brain activity by utilizing the light measurement technology for a living body shown in FIG. 2, the location with the changes in blood flow (quantity) is the upper part of the cerebral cortex on the inner side of the skull. When measuring the blood flow quantity at this location, installing the pair of fibers 4-3 and 4-4 at a point directly above and at the center of this location is known to yield the most sensitivity. This center point is defined as the sampling point and is also defined as the point providing position information for the change in blood flow detected by the pair of sensors. By using the position information on each sampling point to spatially interpolate the change in concentrations of metabolic substances detected by the pair of light sensors, an image can be obtained that shows the changes in levels (concentrations) of metabolic substances within the living body.

Shortening the position intervals between the sampling points is one way to improve the image quality (spatial resolution) of the topographic images obtained by the sensor installation method shown in FIG. 2 for the non-patent document 1. A sensor installation method capable of achieving this placement is disclosed in the patent document 1 and the patent document 2. The sensor installation method shown in the patent document 1 is characterized in that a new sampling point is installed at the center of the four adjacent sampling points, in the sampling point spatial distribution for the sensor installation method shown in FIG. 3. In the patent document 2, the distance between the sampling points is shortened by installing the sensors in a diamond-shape.

[Patent document 1] JP-A No. 178708/2001
[Patent document 2] JP-A No. 121702/2004
[Non-patent document 1] Atsushi Maki, Yuichi Yamashita, Yoshitoshi Ito, Eijyu Watanabe, Yoshiaki Mayanagi, and Hideaki Koizumi, "Spatial and temporal analysis of human motor activity", Medical Physics, Vol. 22 (No. 12), pp. 1997-2005 (1995).
[Non-patent document 2] E. Watanabe, A. Maki, F. Kawaguchi, Y. Yamashita, H. Koizumi, Y. Mayanagi, "Noninvasive Cerebral Blood Volume Measurement During Seizures Using Multichannel Near Infrared Spectroscopic Topography.", Journal of Biomedical Optics, 2000, July, 5 (3), P. 287-290.
[Non-patent document 3] E. Watanabe, A. Maki, F. Kawaguchi, K. Takashiro, Y. Yamashita. H. Koizumi, and Y. Mayanagi, "Non-invasive assessment of language dominance with Near-Infrared Spectroscopic mapping", Neurosci. Lett. 256(1998). [Non-patent document 4] T. Yamamoto, A. Maki, T. Kadoya, Y. Tanikawa, Y. Yamada, E. Okada, and H. Koizumi, "Arranging optical fibers for the spatial resolution improvement of topographical images," Phys. Med. Biol. 47 (2002). [Non-patent document 5] Sandwell, David T, "Biharmonic Spline Interpolation of GEOS-3 and SEASAT Altimeter Data", Geophysical Research Letters, 2, 139-142, 1987.

SUMMARY OF THE INVENTION

The present invention provides a sensor placement method and an imaging method for a living body light measurement system capable of determining the size of the area due to the change in a metabolic substance concentration within a living body. If this method and principle can be developed then the medical treatment field can expand its range of new knowledge. For example this method can reveal whether the brain activation state has recovered after rehabilitation was performed. The size of the brain activation area may for example be small immediately after the patient suffers an ailment but if the size of that area has become larger after appropriate rehabilitation, then the extent of patient recovery can be quantitatively determined if the difference in these sizes can be clearly compared. Moreover, this living body light measurement system possesses a high time resolution compared to fMRI and PET (A maximum image resolution of 10 frames per second can be obtained by the method disclosed in non-patent document 1.). This method therefore also allows observing the change in (area) size resulting from a change in the metabolic substance concentration within the body in the area activated by brain activity. In the technology of the related art, a neurosurgeon for example can acquire moving images of a nervous system seizure. However if the changes over time in the size of the seizure could be observed, then the seizure position and its center position can be estimated with greater precision and the effectiveness for example of neurosurgery enhanced.

The technology of the invention displays images of the difference in size in the activation section. However, in what way the difference in size of a metabolite within the living body is detected using a pair of sensors made up of a pair of light sources and light detectors is described first while referring to the non-patent document 4.

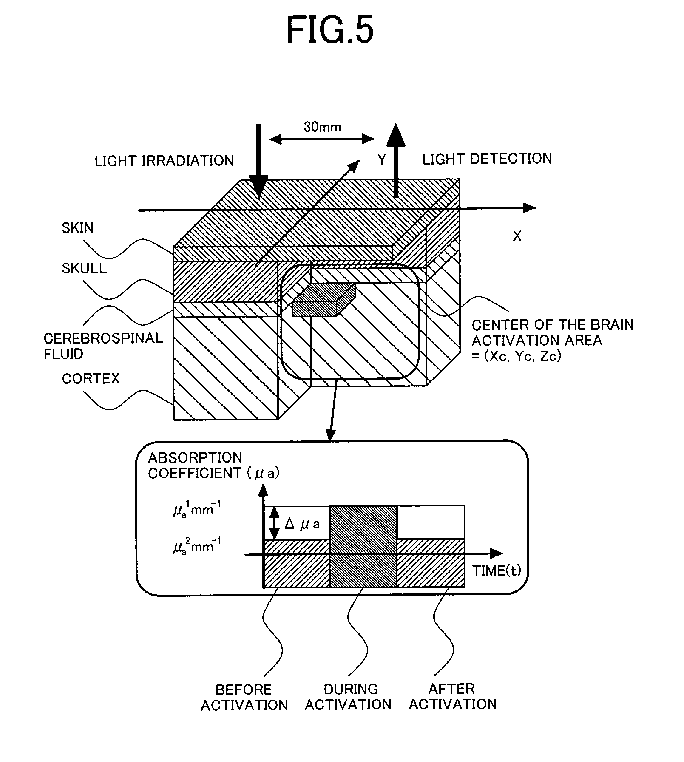

As described in the non-patent document 4, a pair of sensors measures an area where the change in absorption coefficient can be detected within the phantom by utilizing a sample simulating a living body. The method for making this measurement is described using FIG. 5. In FIG. 5, the brain structure is rendered in slab shapes and the cerebral cortex is set as the brain activation area. The area set as the center position is defined as $(X_c, Y_c, Z_c)$. The absorption coefficient ($\mu a$) in this brain activation area is set as $\mu a1$ before and after brain activation, and as $\mu a2$ during the brain activation period. The change in light absorbance detected by the pair of sensors versus the change in the absorbance coefficient is shown as follows:

$$\text{Eq. 1} \qquad \Delta A = -\ln(I1/I2)$$

Here, I1 and I2 are the reflected light intensities that were detected. I1 indicates the value before and after brain activity (in other words, when the absorption coefficient in the brain activation area is μa1). I2 similarly indicates the value during the brain activation period (when the absorption coefficient in the brain activation area is μa2). In the coordinate axes shown in FIG. 5, the ΔA distribution (namely, the function of ΔA supplied by the Xc and Yc variables) when the Xc and Yc positions were changed is called the sensitivity distribution or the spatial distribution of sensitivity, and indicates the area where changes in the absorption characteristic can be detected in the living body.

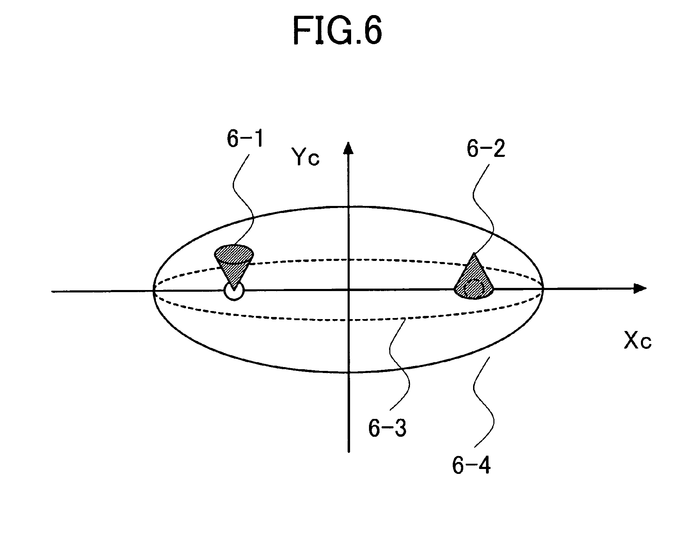

Moreover in the non-patent document 4, that sensitivity distribution is rendered as an elliptical shape. This elliptical shape is known to change when the size of the activation area becomes large (FIG. 6). An elliptical function is utilized to express the shape of this ellipse. This elliptical function is defined as follows.

$$\text{Eq. 2} \qquad \Delta A(x,y) = \exp^{-\frac{x^2}{\Delta x^2}} \exp^{-\frac{y^2}{\Delta y^2}}$$

Here, the Δx and Δy express the FWHM (or full width at half maximum (of the elliptical function).

The reference numerals 6-1 and 6-2 respectively denote the illumination point (light emission position) and the detection point (light detection position). The numerals 6-3 and 6-4 in the figure indicate the respective spatial distribution of sensitivity. The size of the brain activation area per the spatial distribution of sensitivity 6-3 is small compared to the size of the brain activation area per the spatial distribution of sensitivity 6-4. Comparing these two spatial distributions (of sensitivity) shows that even when the size of the brain activation area becomes large, there is virtually no change in the size of the ellipse in the Xc direction. However the size of the ellipse in the Yc direction has become large. These results show that when the size of the brain activation area becomes large, the size of the area where a change in light absorbance becomes detectable with a pair of sensors will expand. A structure for installing (placing) these light detection fibers and light irradiation fibers in order to detect a change in the size of the ellipse was then evaluated. When the area subject to a change in the absorbance coefficient (for metabolic substances) becomes large as shown by the spatial distribution of sensitivity in FIG. 6, the size of the ellipse becomes larger in the Yc direction. Based on these facts, the sampling points (The approximate center point of the sensor pair made up of a light detector and a light source are shown in the figure for purposes of convenience, however the invention is not limited to this method and may include a sensitivity distribution in the range shown in FIG. 6.) were positioned according to the points listed below.

(1) Install multiple sampling points

To construct images showing changes in the metabolic substance concentration within the living body, the changes in the metabolic substance concentration must be detected at multiple locations and an image reconstructed (from the information) based on an imaging algorithm typified by spatial interpolation. Therefore multiple sampling points are installed the same as in the related art.

(2) Install the sampling points of (1) in proximity

In the method of the related art for installing the light detector and a light source (or emitter) fibers, the sampling points are installed at intervals of 21 millimeters. This value is larger than the full width half maximum (FWHM) value for any of the spatial distributions of sensitivity in the y direction shown in non-patent document 4. Therefore installing the multiple sampling points at a closer interval will allow determining the difference in shape in spatial sensitivity distribution (or spatial distribution of sensitivity) that accompanies a change in the area size from fluctuations in blood flow volume.

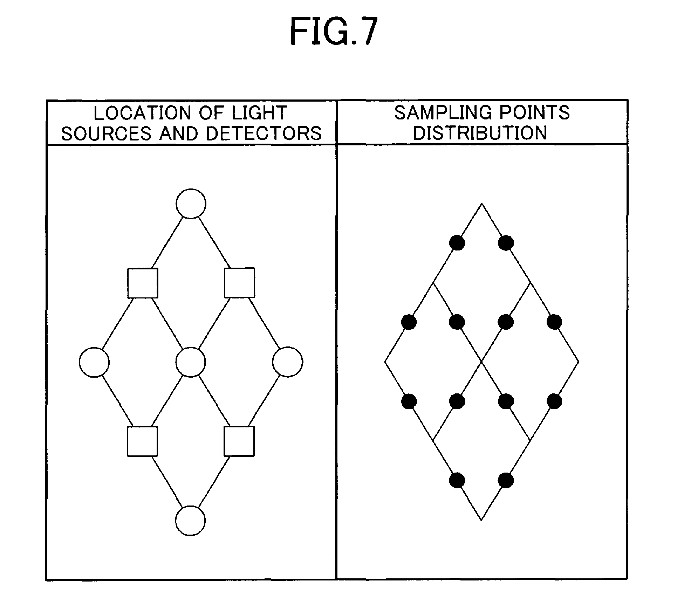

The installation method of the present invention therefore essentially employs a diamond-shaped light source and light detector distribution. In the installation method shown in FIG. 7, the square lattice shape distribution for the light sources and light detectors shown in FIG. 2 is now in an oblique shape. The angle of the lattice installed in a square shape was 90 degrees. However as one example in this invention, the effects from installing a lattice with angles of 60 and 120 degrees are described. The invention is of course not limited to these angle settings. In the installation method shown in FIG. 7, the sampling points are distributed at the peak of rectangles with sides of 15 millimeters and 26 millimeters. The 15 millimeter sampling point interval is about the same when compared with the FWHM (full width at half maximum) value for spatial sensitivity distribution in the Yc direction is the same as disclosed in the non-patent document. Moreover, this value is smaller than the FWHM value in cases where the size of the brain activation region is large; and is larger than the FWHM value in cases where the size of the brain activation area is small. This sampling point interval (15 mm) will therefore allow identifying the size of the activation area.

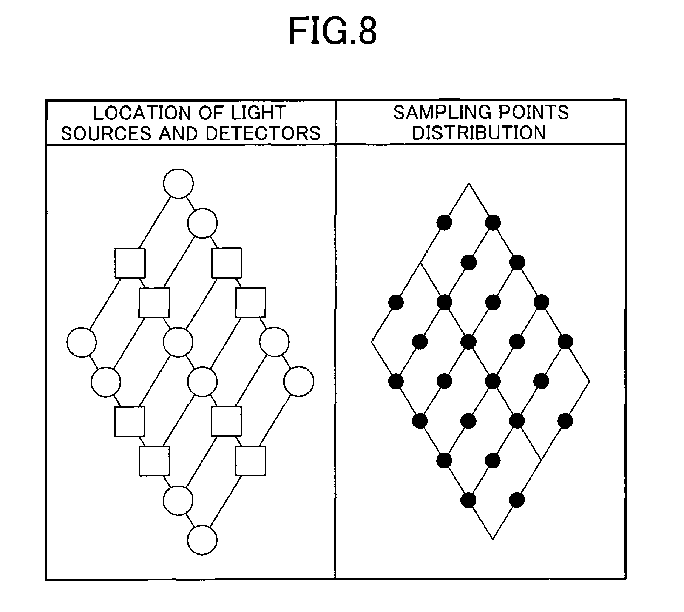

A 26 millimeter sampling interval on the other hand is even larger than the sampling point interval shown for the related art in FIG. 2. In addition to the method shown in FIG. 7 for installing the light sources and light detectors, the method shown in FIG. 8 was also evaluated for installing the light sources and light detectors. In this installation method, another light source and light detector are installed in order to form another sampling point at the center of the four adjacent sampling points shown in FIG. 7. The array installed with a light source and light detector shown in FIG. 7 is the A plane (or A surface), and an array installed with an identical light source and light detector is the B plane. The installation method of FIG. 8 can therefore be achieved by installing another light source or light detector at the center point of the light sources and detectors on the A plane. The installation method shown in FIG. 8 has an installation spacing or interval of 15 millimeters between sampling points. Moreover, all the sampling points are at the peak of the square triangular shape whose length on one side is 15 millimeters.

The present invention renders the effect that differences in size of the brain activation area can be displayed as images. Increased quantities of information can also be acquired from results measuring the concentrations of a metabolic substance in a living body using light. A description is given next while referring to the drawings.

BRIEF DESCRIPTION OF THE DRAWINGS

Fig. 1 shows an algorithm for distinguish brain activation size which is proposed in this invention;
Fig. 2 shows setup for the light measurement system for living body;
Fig. 3 shows spatial distribution of light source, detectors, sampling points for the measurement system that is shown in Fig. 2;
Fig. 4 shows light propagation characteristics in the living body;
Fig. 5 illustrates the simulation method using the phantom (living body structure, optical property, and change in optical property associated with brain activation;
Fig. 6 visualizes the detection of the sensitivity spatial distribution profile by the brain activation size difference;
Fig. 7 shows diamond shaped light source and detectors distribution and spatial sampling point distribution (1);
Fig. 8 shows diamond shaped light source and detectors distribution and spatial sampling point distribution (2);
Fig. 9 shows light sources and detectors distribution for lattice arrangement;
Fig. 10 shows light sources and detectors distribution for diamond shaped arrangement;
Fig. 11 shows topographic images (arrangement of light sources and detectors: Fig. 9);
Fig. 12 shows topographic images (arrangement of light sources and detectors: Fig. 10);
Fig. 13 shows the arrangement of light sources and detectors for diamond shaped distribution (2);
Fig. 14 shows topographic images (arrangement of light sources and detectors: Fig. 13);

Fig. 15 shows the arrangement of light sources and detectors for diamond shaped distribution (3);
Fig. 16 shows topographic images (arrangement of light sources and detectors: Fig. 15);
Fig. 17 shows topographical images display (1);
Fig. 18 shows topographical images display (2); and
Fig. 19 the arrangement of light sources and detectors for diamond shaped distribution (3).

DETAILED DESCRIPTION OF THE PREFERRED EMBODIMENTS

[First Embodiment]

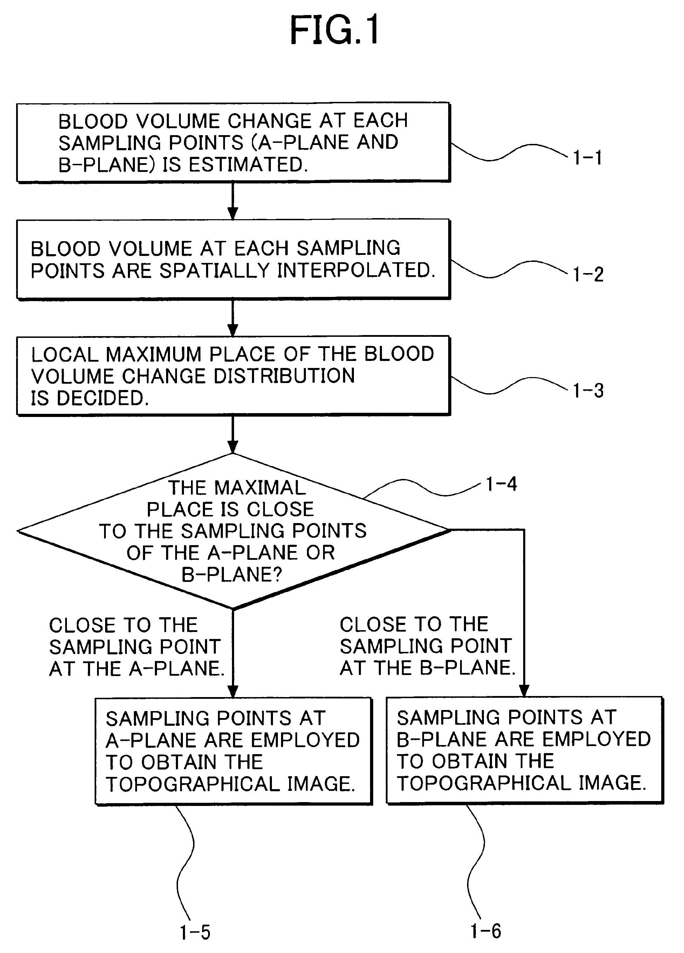

The method for displaying differences in the size of the brain activation area is described next based on the algorithm shown in FIG. 1. First of all, as shown in 1-1, the change in blood volume detected at each of the sampling points on the A plane and B plane are calculated. The installation method for these A and B planes utilizes another light source or light detector installed at the central point of the source-detector pair shown in FIG. 8. A diamond-shaped array is utilized for both the A and B planes. Next, the change in blood flow detected at each sampling point (24 points in the case of FIG. 8) is spatially interpolated (1-2). A desired algorithm may be used for this interpolation but the images obtained by spatial interpolation in the present embodiment utilize an algorithm called the "Inverse Distance" method disclosed in the non-patent document 5. These images obtained by interpolation show the distribution for the change in concentration of metabolic substances in the living body. The location that is the maximum position (place) for blood volume change distribution is determined (1-3). A decision is next made whether the maximum position for blood volume distribution is closer to the A plane or the B plane sampling points (1-4). More specifically, this decision is made by the following described method. The position information on the A plane or B plane sampling points is stored internally in the computer (in this case, combining the processing section and the image display means) shown in 2-6 in FIG. 2.

Next, the maximum position for the blood volume change distribution found by the above described method, and the distances to all the sampling points are calculated. The shortest distance is then selected from these acquired distances. A search is then made for that (shortest distance) sampling point, and whether that sampling point belongs to the A plane or the B plane is calculated. If the maximum position for the blood volume change distribution is nearer to any of the A plane sampling points, then an image is again reconstructed using the A sampling points, and that image is displayed (1-5). If the maximum position for the blood volume change distribution is nearer to any of the B plane sampling points, then an image is again reconstructed using the B sampling points, and that image is displayed (1-6).

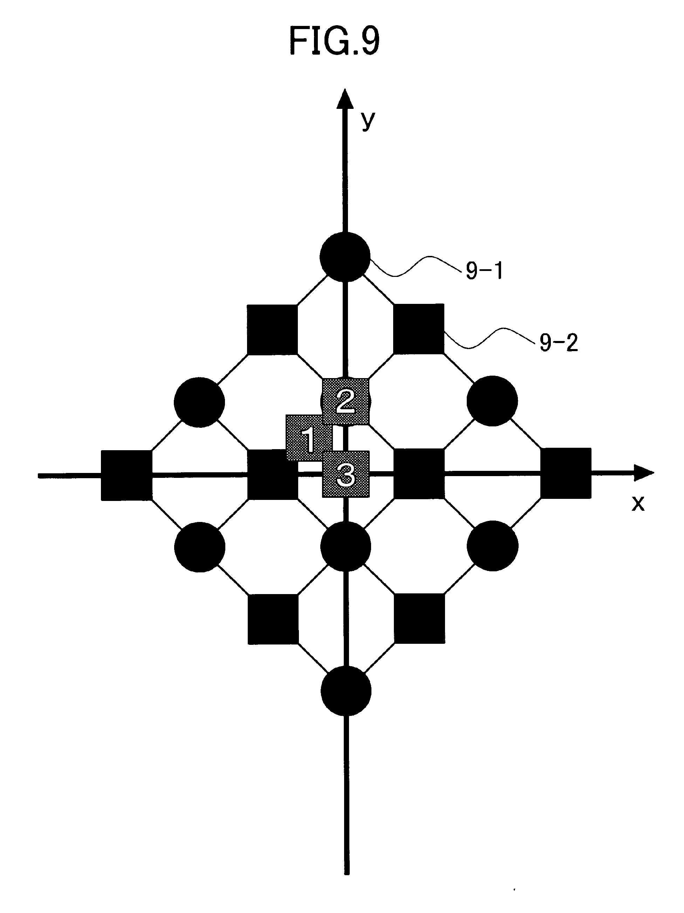

The effectiveness from using diamond-shaped probes is first of all described using FIG. 9, FIG. 10, FIG. 11, and FIG. 12. The drawing in FIG. 9 shows the placement of the lattice. The light sources (9-1) shown by a dot in the figure, and the light detectors (9-2) shown by a black square in the figure are installed at 30 millimeter intervals. Here, X and Y denote the respective coordinate axes. The numerals 1, 2, 3 shown in the figure, denote respective matching positions as follows:

> 1: Center point between the light source and the light detector
> 2: Position directly below the light source or the light detector
> 3: Position in the center of two adjacent light source - light detector pairs

In the present embodiment, a change in concentration (levels) of metabolic substances that accompanies brain activity is assumed to have occurred via these locations 1, 2, 3, and a topographic image is reconstructed by simulation. In this reconstruction method, the size of the brain activation area is first of all assumed, and the spatial (distribution of) sensitivity characteristic supplied by (Eq. 2) is determined. In the embodiment from hereon, the $\Delta x$ and $\Delta y$ shown in (Eq. 2) for the spatial (distribution of) sensitivity characteristic are respectively 15 and 4 (($\Delta x$, $\Delta y$) = (15, 4)) when the size of the brain activation region is small. Similarly, the $\Delta x$ and $\Delta y$ shown in (Eq. 2) for spatial sensitivity are respectively 15 and 13 (($\Delta x$, $\Delta y$) = (15, 13)) when the size of the brain activation area is large. In results from calculating spatial (distribution of) sensitivity characteristic using the phantom body material shown in non-patent document 4, these values are equivalent to a brain activation area with a diameter of approximately 10 millimeters in the former case, and a diameter of approximately 20 millimeters in the latter case.

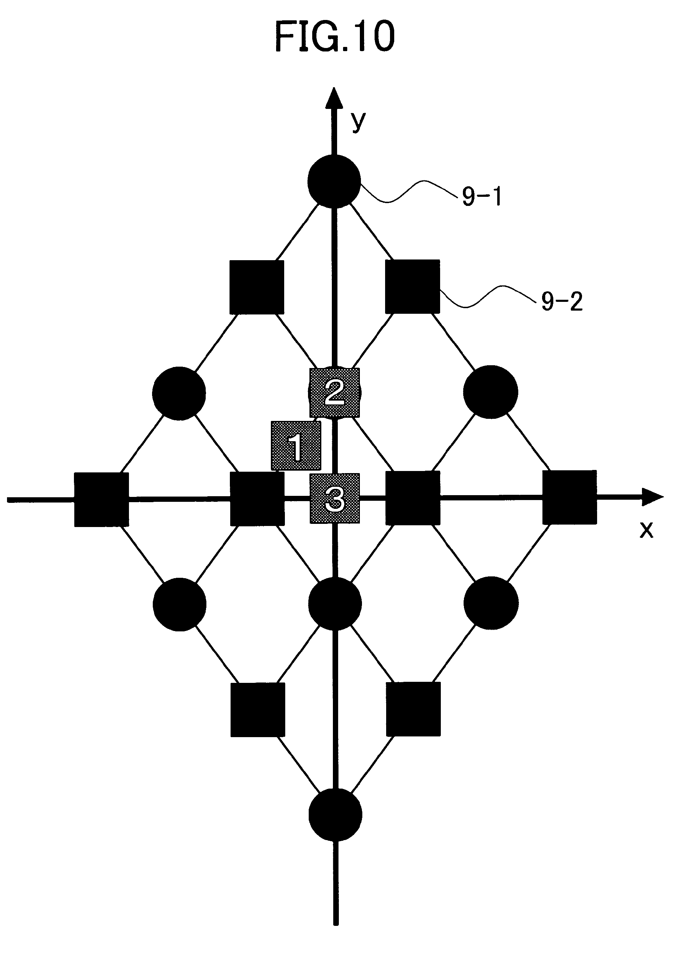

The brain activation position is next set to the 1 or 2 or 3 position. The $\Delta A$ to detect at each sampling point by each pair of light sources-light detectors is determined for each sampling point utilizing (Eq. 2). The interpolation algorithm called the inverse distance method that was disclosed in non-patent document 5 is then used to reconstruct the topographic image. Even the probe installation method shown in FIG. 10 is evaluated by using this same algorithm. FIG. 10 is a drawing showing the probe installation method the same as in FIG. 7. The lattice angles were set to 60 or to 120 degrees. The positions with the numbers 1, 2, 3 in the figure are positions relative to the positions for the numbers (1, 2, 3) shown in FIG. 9 and denote the respective matching positions as follows:

> 1: Center point between the light source and the light detector
> 2: Position directly below the light source or the light detector
> 3: Position in the center of two adjacent light source - light detector pairs

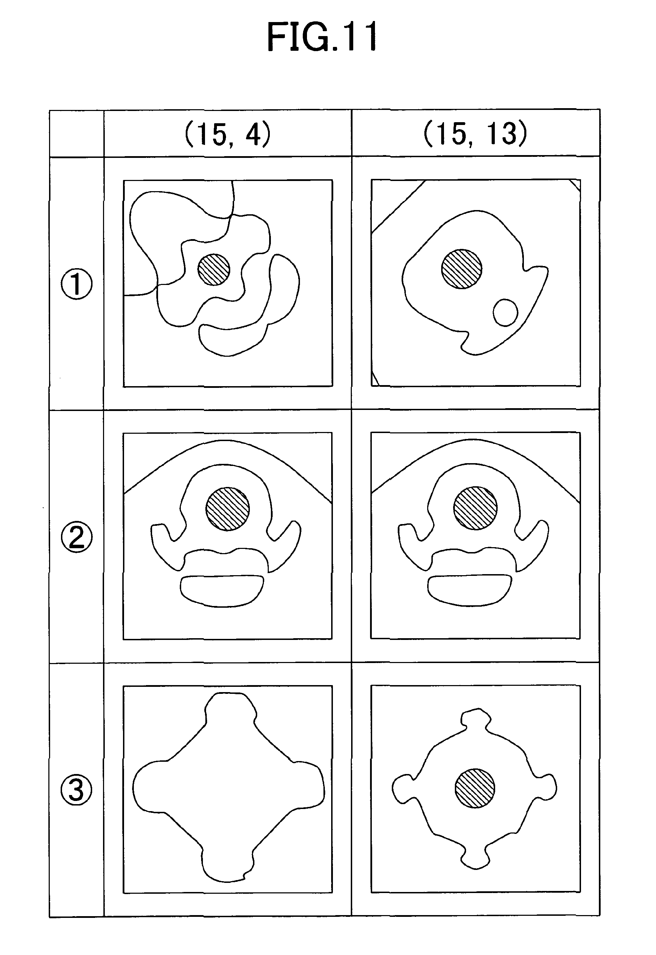

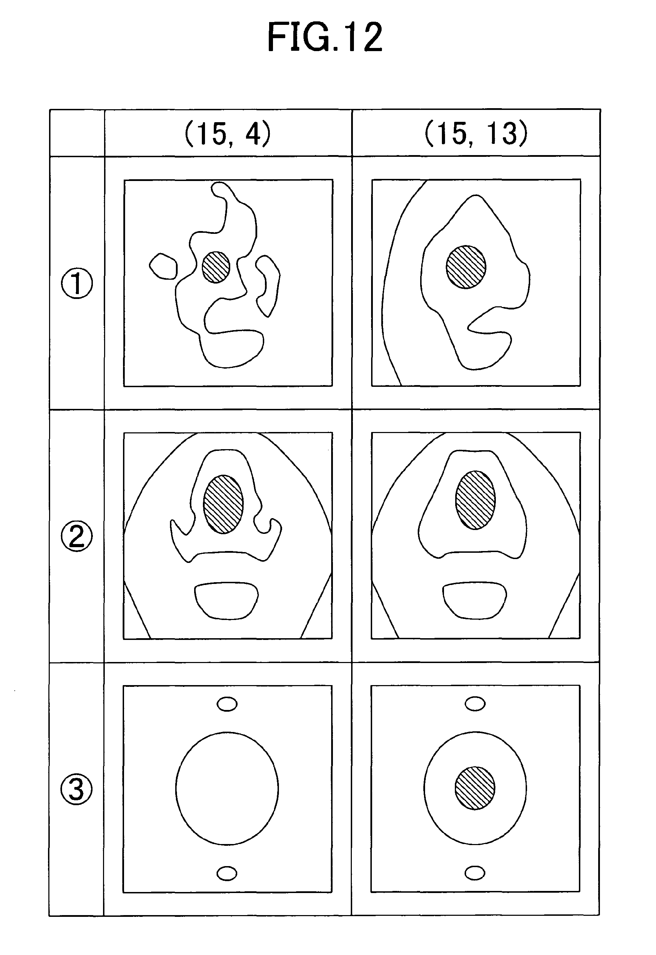

The image quality for the topographic images acquired by the probe installation methods shown in FIG. 9 and FIG. 10 are described while referring to FIG. 11 and FIG. 12. FIG. 11 is a topographic image reconstructed from the lattice position of 90 degrees shown in FIG. 9. The (15, 4) and the (15, 13) in this figure respectively denote the values for the ($\Delta x$, $\Delta y$) shown in (Eq. 2). The image size shown in each table is 160 x 160 millimeters. This area size is larger than the area size for the sampling point distribution at the center point of the light source-light detector pair shown in FIG. 9 and FIG. 10. Even when the area is wider than the sampling point distribution area, a value for the change in blood volume can be estimated by extrapolation from the interpolation method shown in non-patent document 5. In the image shown in the figure, a plus/minus 1 is a standard for the change in blood volume, and this is divided into four equal parts and shown on contour lines. The area shown with the crosshatched lines in each figure indicates areas where that value is 0.5 or more and 1.0 or less. Results clearly shown in FIG. 11 and FIG. 12 are described next.

Results obtained from FIG. 11 are described next. First of all, comparing the surface area formed with crosshatch lines in the location shown in (1) reflects the differences in activation area sizes and a size increase of 1.8 times was obtained. In the location shown in (2) on the other hand, the surface areas with the crosshatch line are nearly the same and a difference in activation area size could not be seen. Further, the image for the location shown in (3) clearly shows that when the size of the brain activation area is small, the brain activity is difficult to detect because the sensitivity distribution size is small. In contrast, the results obtained from FIG. 12 show that the surface area (ratio) of the location shown in (1) is larger than the results from FIG. 11, and that a surface area increase of 2.8 times was obtained. However, in the location shown in (2), the surface area was nearly the same as the results shown in FIG. 11. The image for the area shown in (3) clearly shows that when the size of the brain activation area is small, the brain activity is difficult to detect because the sensitivity distribution size is small. Comparing the probe placements (installations) shown in FIG. 9 and FIG. 10 from the results shown in FIG. 11 and FIG. 12 clearly shows that the diamond-shaped probe can clearly detect differences in the brain activation area size. However, the location that was clearly detected was limited to the location shown in (1) and not the locations in (2) and (3).

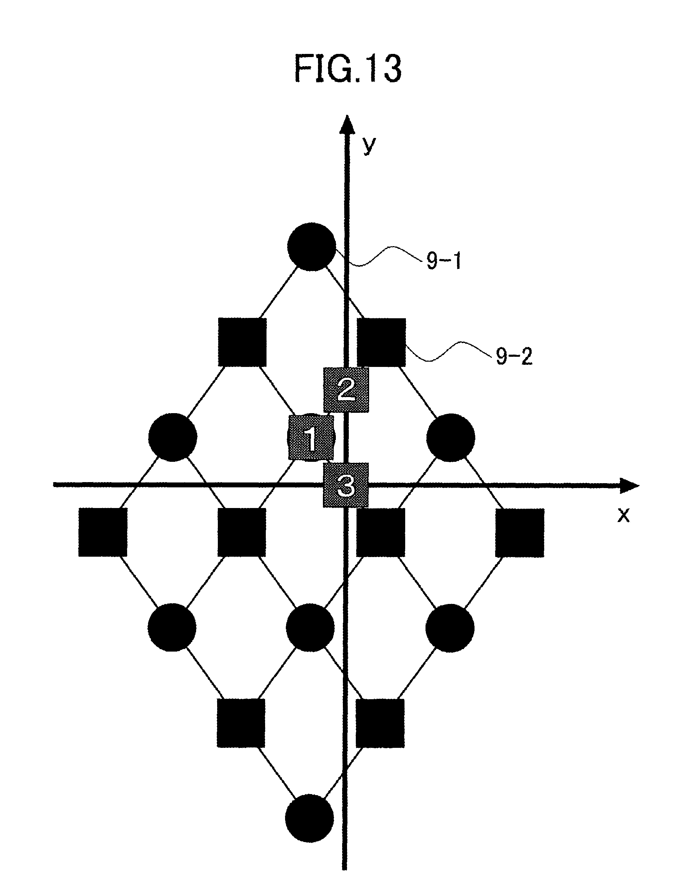

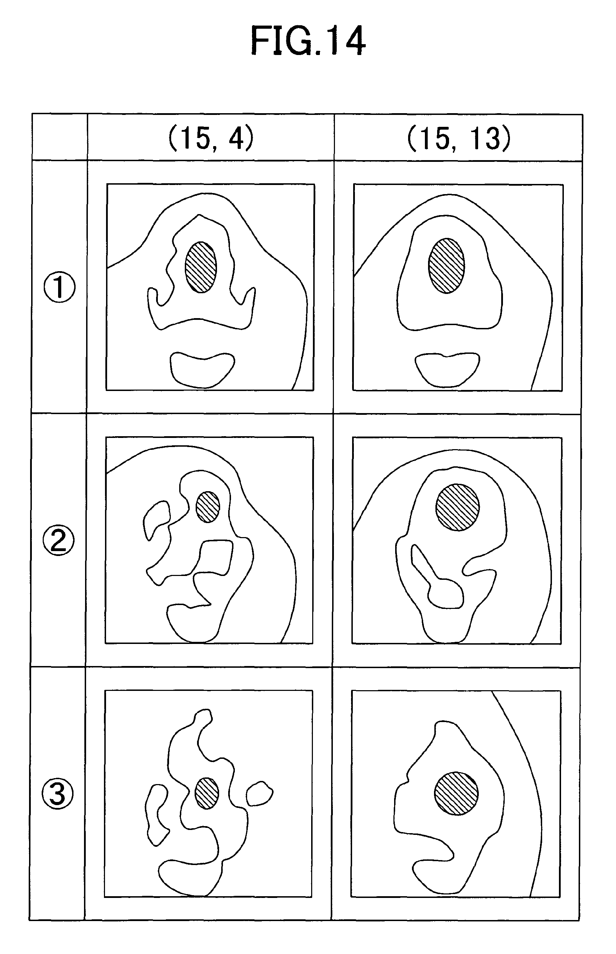

The results obtained from FIG. 9, FIG. 10, FIG. 11, FIG. 12 are next evaluated using FIG. 13 and FIG. 14. Comparing FIG. 10 and FIG. 13 reveals that there are respective deviations of -7.5 millimeters and -13 millimeters in the X and Y directions, in the installation methods for the light source and light detector shown in FIG. 13. Consequently, though the positions 1, 2, 3 in FIG. 10 are:

> 1: Center point between the light source and the light detector
> 2: Position directly below the light source or the light detector
> 3: Position in the center of two adjacent light source - light detector pairs in Fig. 13, the 1, 2, 3 positions have become:

>> 1: Position directly below the light source or the light detector
>> 2: Center point between the light source and the light detector
>> 3: Center point between the light source and the light detector

Results from constructing an image showing the change in blood flow volume are shown in FIG. 14. Here the same algorithm was used as when constructing the images shown in FIG. 11 and FIG. 12. In this figure, the surface area for the crosshatched section in (1) is about 1 time larger. In contrast, the surface areas of the crosshatched sections in (2) and (3) are approximately 2.5 times larger. The locations shown in (2) and (3) have become the center point for the light source and the light detector and so are identical to the location 1 shown in FIG. 10.

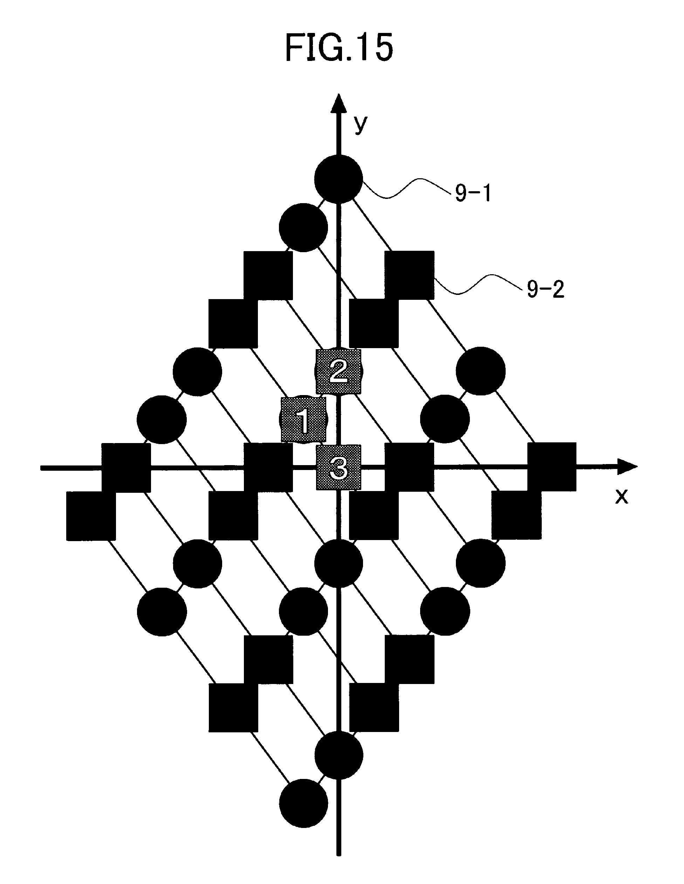

The above simulation results reveal that locations where the difference in brain activation area size can be clearly detected, match brain activation area positions at the sampling point at the center point between the light source and light detector. Here, a topographic image was reconstructed utilizing the light source and light detector installation positions shown in FIG. 15. The locations shown by the numerals 1, 2, 3 in the figure are locations identical to the brain activation area positions shown in FIG. 10 and FIG. 13. These positions are as follows:

> 1: Center point between the light source and the light detector
> 2: Center point between the light source and the light detector
> 3: Center point between the light source and the light detector

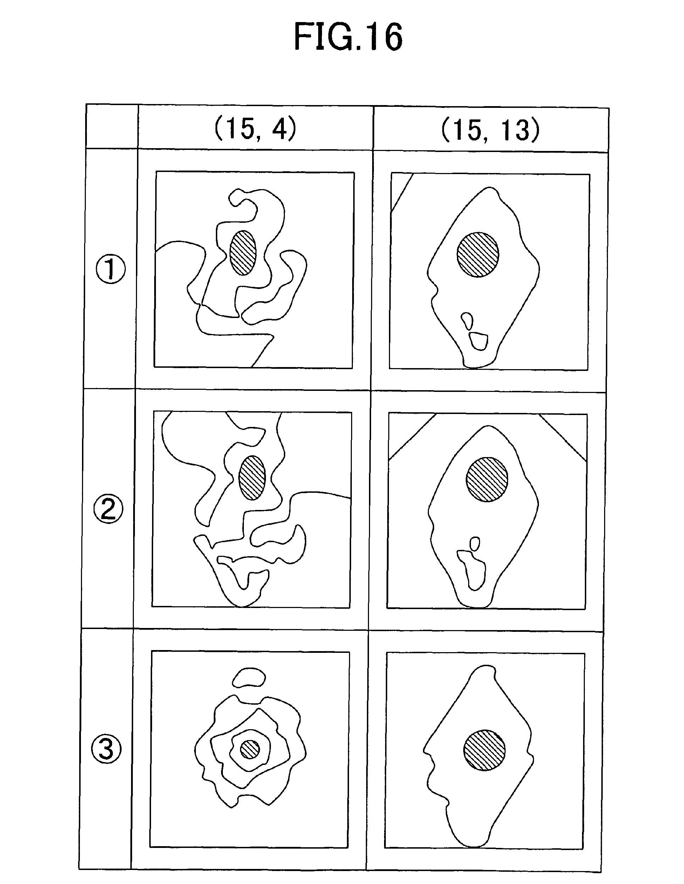

FIG. 16 is a reconstructed image of the brain activation area. In (1) and (2) in this figure, the difference in brain activation area size has increased 1.8 times. However in (3), the area has increased 3.1 times. The locations (1), (2), and (3) are each the center point between the light source and the light detector, however the light source - light detector array is anisotropic as shown in FIG. 15. Consequently, the surface area is different even if the sampling point is the same.

Whereupon as shown in FIG. 1, a decision is made whether the position of the brain activation area is near a sampling point position on the A plane or a sampling points position on the B plane. When decided the position is nearer the sampling points on the A plane, then the image is reconstructed using the sampling points on the A plane. However when the position is nearer the sampling points on the B plane, then the image may be reconstructed using the sampling points on the B plane. Consequently, the difference in the size of the brain activation section can be clearly shown in an image.

[Second Embodiment]

The actual display method is described next based on the image construction method shown in the first embodiment.

Evaluating the results shown up to now in FIG. 12, FIG. 14 and FIG. 16 clearly shows the following conclusions. Firstly, as shown in the first embodiment, in order to display differences in the size of brain activation areas, the change in blood flow volume is calculated at all the sampling points, spatial interpolation is performed using position information on all the sampling points, the position comprising the maximum location for change in blood flow volume is determined from the interpolation results, whether that maximum location is closer to the A plane or the B plane sampling points is decided, and if based on those decision results an image is reconstructed using only the sampling points on that plane, then an image is obtained that clearly identifies the size difference in the brain activation area. Secondly, brain activity that could not be detected in FIG. 10 can be detected by utilizing the method for installing the light sources and light detectors shown in FIG. 15. More specifically, when the size of the brain activation area is small $(\Delta x, \Delta y) = (15, 4)$ as in FIG. 12, then the change in blood flow volume in the location (3) that accompanies brain activity cannot be clearly shown in an image. However an image of brain activity can be shown for locations as in FIG. 16, where the size of the brain activation area is the same. These results show that the method in FIG. 15 for installing light detectors and light sources is appropriate when the brain activation area is small.

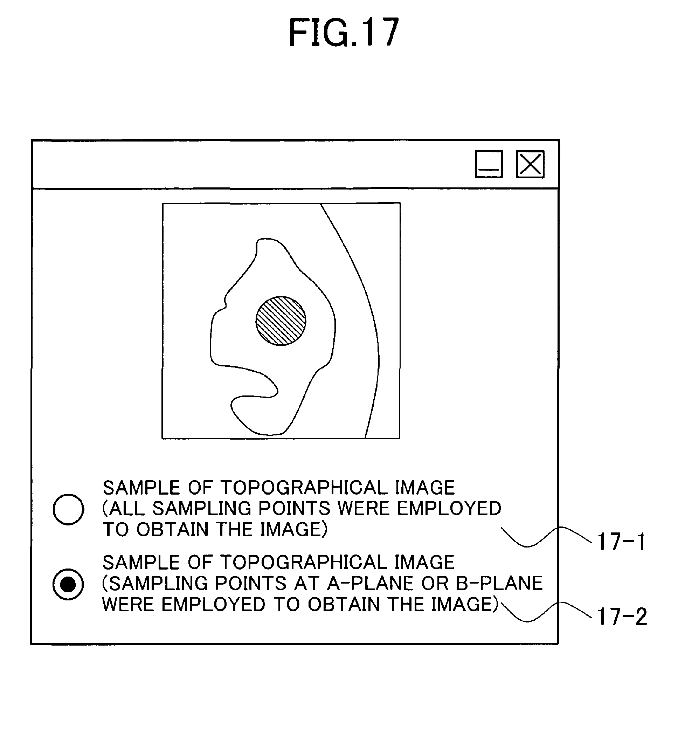

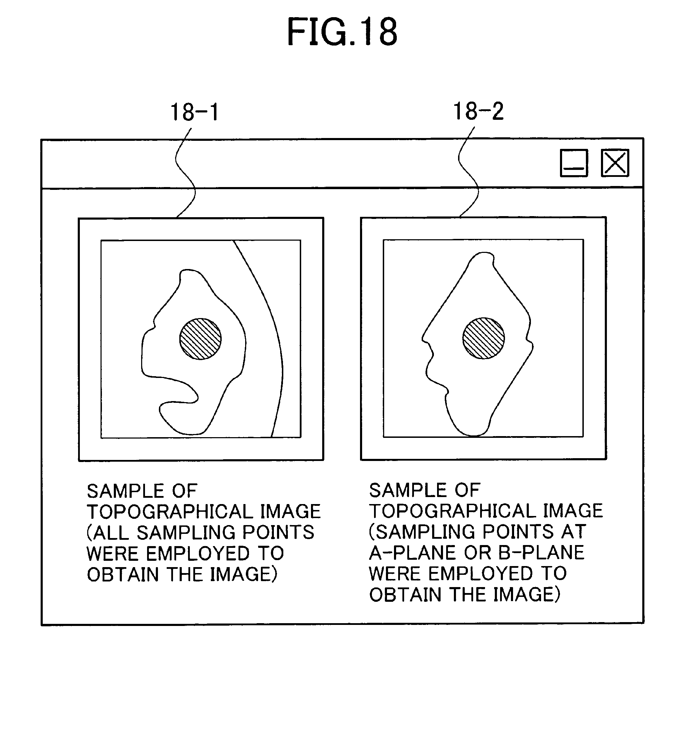

The above results confirm that the two image construction methods have both advantages and disadvantages. FIG. 17 and FIG. 18 show methods for displaying reconstructed images that can utilize those advantages and disadvantages. In FIG. 17, a switch is capable of selecting either "Use all sampling points to reconstruct image" (17-1) or "Use any of the sampling points on the A plane or the B plane to reconstruct the image" (17-2). FIG. 18 shows an image obtained by using all sampling points to reconstruct the image (18-1), and an image obtained using either the A plane or the B plane sampling points to reconstruct the image (18-2). These results show that the advantages and disadvantages of the image construction methods can be combined in a complementary image construction method.

[Third Embodiment]

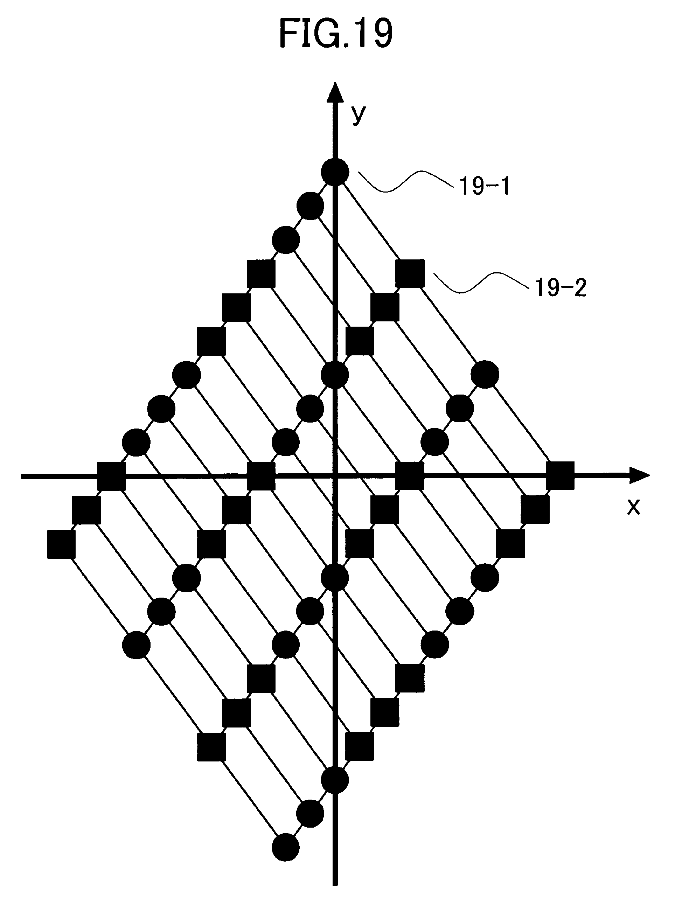

The third embodiment is a variation based on the image construction methods shown in the first embodiment and the second embodiment. The embodiment shown in FIG. 15 is characterized in that the diamond-shaped sensors of FIG. 10 are installed in sets of two each. However these sets are not limited to two each and sets containing multiple sensors are no problem whatsoever. The example shown in the variation shown in FIG. 19 utilizes sets of three sensors each. Utilizing this type of installation method allows increasing the number of sampling points. Therefore by providing sampling points at positions near the maximum place (point) of brain activity, the size of the brain activation area can be clearly identified as disclosed in the first embodiment.

The present invention is capable of clearly detecting differences in the brain activation area size and can therefore quantitatively evaluate the recovery of brain functions due to rehabilitation or other factors. This quantitative evaluation of brain functions can be utilized in fields such as medical treatment, welfare, and education.

**Claims**

1. A measurement method utilized in a light measurement device for a living body to irradiate light onto a subject, receive the light propagated from within the subject, and measure a fluctuation in the metabolic substance concentration within the subject, the method comprising:

   a first step for installing multiple first and a second sensor arrays including two or more light sources and two or more light detectors on the subject;
   a second step for calculating the maximum position of the fluctuation in the metabolic substance concentration measured by the first and second sensor arrays;
   a third step for calculating whether the maximum position is nearer the sampling point of the first sensor array or that of the second sensor array; and
   a fourth step for measuring a change in the metabolic substance concentration by utilizing the first or second sensor arrays including a sampling point calculated to be in proximity to the maximum position of the third step.

2. The method of claim 1, further including a step for selecting the first sensor array or the second sensor array or both the first and second sensor arrays for measuring the fluctuation in the concentration of the metabolic substances.

3. The method of claim 1, including a fifth step for making an image of the distribution in the change in the metabolic substance concentration from the change in the metabolic substance concentration acquired in the fourth step.

4. A light measurement device for a living body comprising:

   a light source to irradiate light on the subject;
   a light detector to detect light that propagated through the subject;
   a first and a second sensor array including multiple light sources and multiple light detectors; and
   processing means for processing a change in the metabolic substance concentration within the subject from the light detected by the light detector, wherein the processing means calculates the maximum position of the change in metabolic substance concentration measured by the first and second sensor arrays, and calculates whether the maximum position is nearer the sampling point of the first sensor array or that of the second sensor array, and calculates the change in the metabolic substance concentration by utilizing the first or the second sensor array including a sampling point calculated to be in proximity to the maximum position.

5. The device of claim 4, comprising image display means to show the distribution of the change in metabolic substance concentration.

6. The subject matter of claim 1 or 4, wherein the first and second sensor arrays include multiple light sources and multiple light detectors installed at alternate positions in a lattice shape.

7. The subject matter of claim 6, wherein the light sources and the light detectors are installed respectively equivalent distances.

8. The subject matter of claim 6, wherein the lattice is a diamond shape.

9. The subject matter of claim 6, wherein the first sensor array is installed so that the first sensor array light sources or the light detectors are installed on the sampling points of the second sensor array.

10. The subject matter of claim 1 or 4, wherein the sampling point is the approximate center point between the light source and the light detector.

# FIG.1

BLOOD VOLUME CHANGE AT EACH SAMPLING POINTS (A-PLANE AND B-PLANE) IS ESTIMATED. — 1-1

↓

BLOOD VOLUME AT EACH SAMPLING POINTS ARE SPATIALLY INTERPOLATED. — 1-2

↓

LOCAL MAXIMUM PLACE OF THE BLOOD VOLUME CHANGE DISTRIBUTION IS DECIDED. — 1-3

↓

THE MAXIMAL PLACE IS CLOSE TO THE SAMPLING POINTS OF THE A-PLANE OR B-PLANE? — 1-4

CLOSE TO THE SAMPLING POINT AT THE A-PLANE.　　　　CLOSE TO THE SAMPLING POINT AT THE B-PLANE.

SAMPLING POINTS AT A-PLANE ARE EMPLOYED TO OBTAIN THE TOPOGRAPHICAL IMAGE.

SAMPLING POINTS AT B-PLANE ARE EMPLOYED TO OBTAIN THE TOPOGRAPHICAL IMAGE.

1-5　　　　1-6

# FIG.2

# FIG.3

| LIGHT SOURCE AND DETECTOR DISTRIBUTION | SAMPLING POINTS DISTRIBUTION |
|---|---|

30 mm

3-1

3-2

21 mm

3-3

# FIG.4

4-3

4-4

4-1

4-2

AREA

# FIG.5

30mm

LIGHT IRRADIATION

Y

LIGHT DETECTION

SKIN

SKULL

X

CEREBROSPINAL
FLUID

CENTER OF THE BRAIN
ACTIVATION AREA
= (Xc, Yc, Zc)

CORTEX

ABSORPTION
COEFFICIENT ($\mu$a)

$\mu_a^1 mm^{-1}$

$\Delta \mu a$

$\mu_a^2 mm^{-1}$

TIME(t)

BEFORE
ACTIVATION

DURING
ACTIVATION

AFTER
ACTIVATION

# FIG.6

# FIG.7

| LOCATION OF LIGHT SOURCES AND DETECTORS | SAMPLING POINTS DISTRIBUTION |
|---|---|

# FIG.8

| LOCATION OF LIGHT SOURCES AND DETECTORS | SAMPLING POINTS DISTRIBUTION |
|---|---|
| | |

# FIG.9

# FIG.10

# FIG.11

# FIG.12

## FIG.13

# FIG.14

# FIG.15

# FIG.16

# FIG.17

○ SAMPLE OF TOPOGRAPHICAL IMAGE
(ALL SAMPLING POINTS WERE EMPLOYED
TO OBTAIN THE IMAGE)                    17-1

◉ SAMPLE OF TOPOGRAPHICAL IMAGE
(SAMPLING POINTS AT A-PLANE OR B-PLANE
WERE EMPLOYED TO OBTAIN THE IMAGE)      17-2

# FIG.18

18-1                    18-2

SAMPLE OF
TOPOGRAPHICAL IMAGE
(ALL SAMPLING POINTS
WERE EMPLOYED TO
OBTAIN THE IMAGE)

SAMPLE OF
TOPOGRAPHICAL IMAGE
(SAMPLING POINTS AT
A-PLANE OR B-PLANE
WERE EMPLOYED TO
OBTAIN THE IMAGE)

# FIG.19

**European Patent Office**

**EUROPEAN SEARCH REPORT**

Application Number

EP 05 00 1807

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | EP 1 245 192 A (HITACHI, LTD; HITACHI MEDICAL CORPORATION) 2 October 2002 (2002-10-02) * paragraphs [0018] - [0022]; claim 1; figure 1 * ----- | 1-10 | A61B5/00 A61B10/00 |
| A | US 5 853 370 A (CHANCE ET AL) 29 December 1998 (1998-12-29) * column 6, line 30- - column 8, line 35; claim 1 * ----- | 1-10 | |
| A | WO 01/24691 A (INSTRUMENTARIUM CORPORATION) 12 April 2001 (2001-04-12) * page 3, line 24 - page 5, line 30; claim 1 * ----- | 1-10 | |
| A | EP 1 374 778 A (HITACHI, LTD; HITACHI MEDICAL CORPORATION) 2 January 2004 (2004-01-02) * the whole document * ----- | 1-10 | |

TECHNICAL FIELDS SEARCHED (Int.Cl.7)

A61B

The present search report has been drawn up for all claims

| Place of search | Date of completion of the search | Examiner |
|---|---|---|
| Munich | 1 April 2005 | Chopinaud, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 00 1807

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2005

| Patent document cited in search report | | Publication date | Patent family member(s) | | Publication date |
|---|---|---|---|---|---|
| EP 1245192 | A | 02-10-2002 | JP | 2001178708 A | 03-07-2001 |
| | | | EP | 1245192 A1 | 02-10-2002 |
| | | | CN | 1413097 A | 23-04-2003 |
| | | | WO | 0147422 A1 | 05-07-2001 |
| | | | US | 2003088162 A1 | 08-05-2003 |
| US 5853370 | A | 29-12-1998 | AU | 4349097 A | 02-04-1998 |
| | | | DE | 69728408 D1 | 06-05-2004 |
| | | | DE | 69728408 T2 | 23-12-2004 |
| | | | EP | 0926981 A1 | 07-07-1999 |
| | | | WO | 9810698 A1 | 19-03-1998 |
| | | | US | 6397099 B1 | 28-05-2002 |
| | | | US | 2004064052 A1 | 01-04-2004 |
| WO 0124691 | A | 12-04-2001 | AU | 7440500 A | 10-05-2001 |
| | | | EP | 1135053 A1 | 26-09-2001 |
| | | | WO | 0124691 A1 | 12-04-2001 |
| | | | US | 6526297 B1 | 25-02-2003 |
| EP 1374778 | A | 02-01-2004 | JP | 2002291751 A | 08-10-2002 |
| | | | EP | 1374778 A1 | 02-01-2004 |
| | | | US | 2004054271 A1 | 18-03-2004 |
| | | | WO | 02080777 A1 | 17-10-2002 |

For more details about this annex : see Official Journal of the European Patent Office, No. 12/82